# EUROPÄISCHE PATENTSCHRIFT

(11) **EP 0 544 075 B1**
(45) Veröffentlichungstag und Bekanntmachung des Hinweises auf die Patenterteilung: **29.11.1995**
(21) Anmeldenummer: 92116512.2
(22) Anmeldetag: 26.09.1992
(51) Int. Cl.: G01N 25/56, G01N 33/02

(54) **Anordnung zur Überwachung der Umgebungsfeuchte gelagerter oder transportierter, feuchteempfindlicher Produkte.**
System of monitoring the ambient humidity of stored or transported, humidity-sensitive products.
Système de contrôle de l'humidité ambiante de produits supportés ou transportés, sensibles à l'humidité.

(30) Priorität: 09.10.1991 DE 4133414
(43) Veröffentlichungstag der Anmeldung: 02.06.1993
(73) Patentinhaber: G + H MONTAGE GmbH, D-67059 Ludwigshafen (DE); DEKA SENSOR + TECHNOLOGIE INGENIEURBÜRO GbRmbH Wernecke, Wonneberger, Zehner, D-14513 Teltow (DE)
(72) Erfinder: Idler, Lothar, W-2050 Hamburg 80 (DE); Oberacker, Rolf, W-2000 Hamburg 13 (DE); Beckervordersandtforth, Kurt, W-1000 Berlin 13 (DE); Wernecke, Roland, Dr., O-1599 Potsdam (DE); Wonneberger, Reiner, O-1533 Stahnsdorf (DE); Zehner, Christian, Dr., O-1532 Kleinmachnow (DE)
(74) Vertreter: Fleck, Thomas, Dr. Dipl.-Chem.

(56) Entgegenhaltungen:
- DE-A- 1 953 866
- US-H- 381
- PATENT ABSTRACTS OF JAPAN vol. 9, no. 9 (P-327) ; & JP-A-59 157 565
- 32ND ELECTRONIC COMPONENTS CONFERENCE 10. Mai 1982, SAN DIEGO, CA, USA, Seiten 406-409; F.R. MOSER, H.C. BARON "Moisture measurements in IBM circuit packages"

## Beschreibung

Die Erfindung betrifft eine Anordnung zur Überwachung der Umgebungsfeuchte nach dem Oberbegriff des Anspruches 1.

Eine derartige Anordnung ist aus der US-H-H381 bekannt.

Die dortigen Ausführungen beziehen sich jedoch auf "sealed" oder "enclosed container", insbesondere auf Container, die Projektile darstellen und mit explosivem Material gefüllt sind. Die "Produkte" sind keinesfalls nochmals verpackt . Großraumcontainer, insbesondere solche, die in gestapelter Form auf Schiffen für eine längere Reisezeit zum Transport verderblicher Güter, wie Lebensmittel, eingesetzt werden, werden dort mit keinem Wort erwähnt. Die Lagerumgebungsluft in solchen Containern ist jedoch von großer Bedeutung, da es zahlreiche feuchteempfindliche Produkte und Erzeugnisse gibt, bei denen besonders beim Transport und bei der Lagerung zur Vermeidung von Qualitätsminderungen und sonstigen Schäden sichergestellt sein muß, daß die Umgebungsatmosphäre eine bestimmte maximale Luftfeuchtigkeitsgrenze nicht überschreitet.

Es gibt zahllose unterschiedlichste Erzeugnisse, für die dies gilt; von landwirtschaftlichen und allgemein organischen Produkten, bei denen hohe Umgebungsfeuchte zu Fäulnis führt, bis hin zu industriellen Erzeugnissen und Geräten, bei denen im Ergebnis unzulässige Umgebungsfeuchte, Permeation, Korrosionsschäden und dergleichen auftreten.

Solche feuchteempfindlichen Produkte werden üblicherweise in geschlossenen Systemen oder Verpackungssystemen transportiert, die mindestens aus einer äußeren, formstabilen und mechanischen Umhüllung oder Gehäuse, einem Container oder einer Kiste etwa, und ggf. einer inneren Verpackung in der Art einer Folie, beispielsweise aus Aluminium oder Polyethylen, bestehen.

Sofern ohne Verpackungsfolie eine Lagerung oder ein Transport von Produkten durchgeführt werden kann, hat man bisher in Kauf genommen, daß ein Teil der Produkte, verdorben oder sonstwie beschädigt, entsorgt werden musste.

Zwar hat man erkannt, daß die Lagerumgebungsluft in Schiffs-Containern von Bedeutung ist, um die Lagerzeit der Produkte ausdehnen zu können und so den Verbraucherwünschen besser gerecht zu werden, jedoch davon Abstand genommen, die Umgebungsfeuchte als einen der verantwortlichen Parameter zu überwachen oder gar zu steuern. Unter anderem wurde dieses auf das große Volumen und auf die mit zunehmendem Arbeitsvolumen steigende Fehlerquote zurückgeführt. Auch hat man darüberhinaus bei einer Vielzahl von Containern ein erhöhtes Ausmaß an Gasdichtigkeitsproblemen,
Sofern Verpackungsfolien eingesetzt werden, werden diese häufig luftdicht verschweißt; wo dies - beispielsweise wegen der Größe oder Form des Objektes - nicht möglich ist, werden die Folien auf alle Fälle so eingeschlagen, daß ein möglichst geringer Eintritt der Umgebungsfeuchte möglich ist.

Es ist weiterhin bekannt, im Inneren der Folien sog. "Trockenbeutel" aus hygroskopischem Material anzuordnen, so daß die die verpackten Produkte umgebende Luftfeuchtigkeit eine gewisse Zeit unterhalb einer kritischen Grenze gehalten werden kann.

Die Anzahl der zu verwendenden Trockenbeutel wird zwar vom Hersteller oder Transporteur, bezogen auf das Verpackungsvolumen, angegeben, jedoch ist eine laufende Kontrolle der tatsächlichen Luftfeuchtigkeit im Inneren der Verpackung nicht möglich.

Der für die verpackten Produkte kritische Luftfeuchtewert kann sowohl durch die Sättigung des hygroskopischen Trockenmittels als auch einen Defekt der äußeren und/oder inneren Verpackung überschritten werden; das gleiche kann durch extreme Witterungs- oder Klimaänderungen bewirkt werden. Eine Kontrolle ist - wie ausgeführt - nach dem Stand der Technik nicht möglich, allenfalls können einzelne Verpackungseinheiten stichprobenartig mit Meßsonden o.ä. kontrolliert werden, wobei dazu die innere Verpackung geöffnet, d.h. verletzt werden muß, was einen zusätzlichen Aufwand zum Wiederverschließen bedingt. Dabei ist noch zu berücksichtigen, daß Folienschweißgeräte o.ä. zum Wiederverschließen der inneren Verpackung bei Unterwegs-Kontrollen üblicherweise nicht zur Verfügung stehen werden.

Aufgabe der Erfindung ist es demnach, eine Anordnung zur Feuchteüberwachung besonders sorgfältig verpackter, gelagerter oder transportierter Produkte anzugeben, die sich auch bei gestapelten Schiffscontainern anwenden lässt und keine Eingriffe in das System oder die Verpackung erfordert, d.h. schnell, einfach und zerstörungsfrei die entsprechenden Informationen ausserhalb vom System liefert, und weiterhin eine Fernüberwachung und Einflussnahme auf die Umgebungsfeuchtigkeit der gefährdeten Produkte gestattet. Hierzu gehört auch die sichere Befestigung der Sensoren, die trotz der Verpackung der produkte genaue Meßwerte liefern soll.

Gelöst wird diese Aufgabe durch die Merkmale des kennzeichnenden Teiles des Patentanspruches 1. Die Unteransprüche beinhalten bevorzugte Ausgestaltungen der Erfindung.

Der mit der Erfindung erzielte Vorteil liegt dabei insbesondere darin, daß eine kontinuierliche oder auch sporadische Kontrolle der gefährdete verpackte Produkte umgebenden Umgebungsfeuchtigkeit (Luft oder andere Gase oder Gasgemische) auch bei gestapelten Schiffscontainern jederzeit möglich ist, ohne daß in das System eingegriffen, d.h. dieses geöffnet, verletzt oder zerstört werden muss.

Ein weiterer Vorteil liegt darin, daß das an das Äussere des Systems geleitete elektrische Signal als Maß der Luftfeuchtigkeit im Inneren sowohl zur Anzeige vor Ort dienen kann als auch in bekannte Netze zur Fernübertragung, Telefon- oder Cityrufnetze etwa, einspeisbar ist und damit auch eine Fernüberwachung gestattet oder zu Steuer- und Regelzwecken eingesetzt werden kann.

Das elektrische Signal kann sowohl als kontinuierliches Überwachungssignal ausgewertet und analog oder quasianalog als Feuchtewert oder -bereich angezeigt werden als auch zur Schwellwertanzeige, z.B. zur optischen und/oder akustischen Alarmauslösung, dienen.

Die Erfindung soll nachstehend beispielhaft näher erläutert werden.

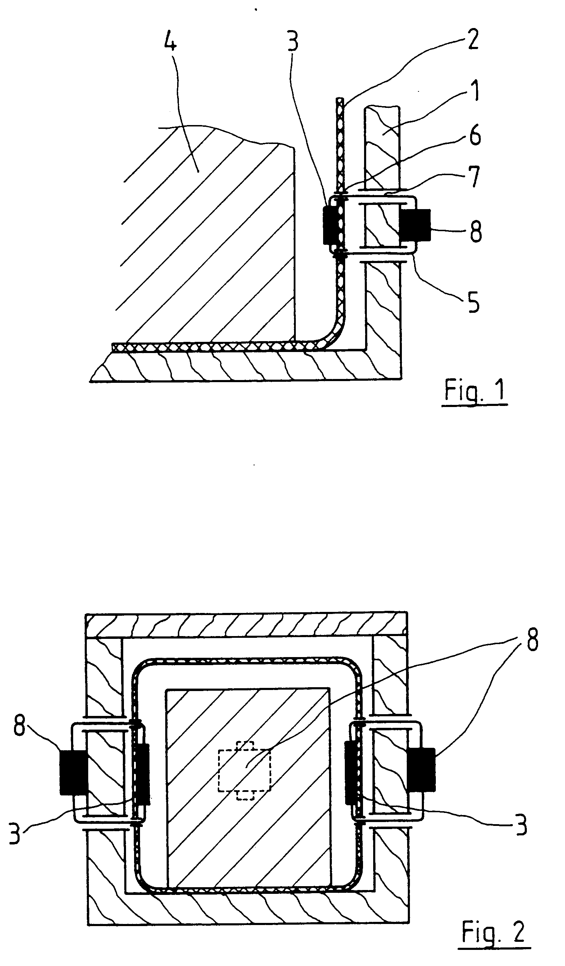

Erfindungsgemäß wird eine Verpackungsfolie für das Produkt verwendet und ein Feuchtesensor 3, wie in Fig. 1 gezeigt, ist einfach im Inneren eines aus einer Kiste 1 und einer inneren Folie 2 bestehenden Verpackungssystems angeordnet. Er mißt die ein feuchteempfindliches, verpacktes Produkt 4 umgebende Luftfeuchte auf elektrischem Wege; die elektrischen Anschlüsse 5 werden durch das Verpackungssystem mittels innerer und äußerer Durchführungen 6,7 nach außen geführt und dort an ein Meßgerät 8, das die Information akustisch und/oder optisch anzeigt und gleichzeitig eine Batterie zur Lieferung der für den Feuchtesensor erforderlichen Versorgungsspannung aufweist, geführt.

Fig. 2 zeigt eine ähnliche Anordnung, bei der sich jedoch Meßgeräte 8 an vier Außenflächen der Kiste 1 befinden, so daß auch bei willkürlicher Lager- und Stapelweise der Kisten 1 die Zugänglichkeit erhalten bleibt. Bei einer solchen Anordnung wird man natürlich zur Vermeidung von Beschädigungen die Meßgeräte 8 in die Oberfläche der Kiste 1 einlassen. Dabei kann ein einzelner Feuchtesensor 3 auf alle Meßgeräte 8 wirken; es kann auch jedem Meßgerät 8 ein separater Feuchtesensor 3 im Inneren zugeordnet werden.

Die Anzeige am Meßgerät 8 wird zweckmäßigerweise als LCD- oder LED-Anzeige ausgeführt, der Ladungszustand der zur Versorgung des Feuchtesensors 3 erforderlichen Batterien kann ebenfalls angezeigt werden.

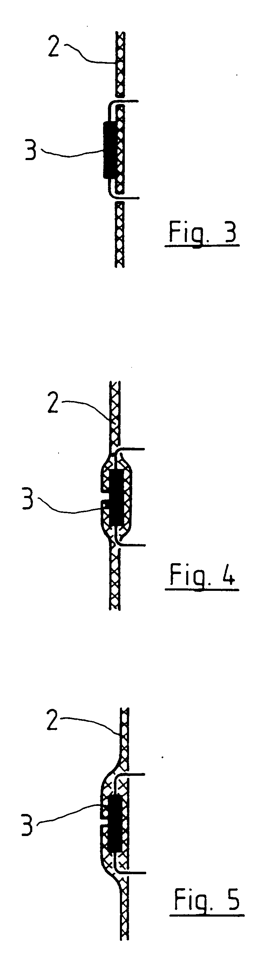

Fig. 3 zeigt eine Verpackungsfolie 2, an der Feuchtesensoren 3 einerseits befestigt sind; Fig. 4 und Fig. 5 jeweils eine solche Folie 2, bei der Feuchtesensoren 3 eingelagert sind.

In all diesen Fällen werden die elektrischen Anschlüsse mittels Durchführungen 6 aus der Folie 2 herausgeführt.

Die Befestigung der Feuchtesensoren 3 an der Folie 2 verhindert, daß der Feuchtesensor 3 beim Transport seine Lage verändert und evtl. durch das feuchteempfindliche Produkt 4 beschädigt werden könnte.

Die Einlagerung der Feuchtesensoren 3 in die Folie 2 ermöglicht darüberhinaus ein schnelles und unkompliziertes Verpacken mit einer vorkonfektionierten Folie 2.

Besonders geeignet als Feuchtesensoren 3 sind dabei beispielsweise die aus den DE-PS 37 34 582, 37 34 608 und 37 34 614 bekanntgewordenen flexiblen, folienartigen Sensorenelemente, die sich geradezu ideal an die Folie 2 anpassen und zudem relativ klein und sehr dünn sind.

Die Erfindung ist aber nicht auf die Anwendung solcher Feuchtesensoren beschränkt.

Es wurde bereits erwähnt, daß zur Spannungsversorgung der Feuchtesensoren 3 Batterien, Akkus oder dgl. erforderlich sind. Diese - in den Figuren nicht näher dargestellten - Energiequellen, sind zweckmäßigerweise mit dem an der Außenseite der Verpackungseinheit angeordneten Meßgerät 8 räumlich integriert, um bei Bedarf ohne weiteres von außen ausgewechselt werden zu können.

Ebenfalls ist es zweckmäßig, die elektronische Signalverarbeitungseinheit, die erforderlich ist, um das von den Feuchtesensoren 3 feuchteäquivalente elektrische Signal in eine optische und/oder akustische Information umzusetzen, ebenfalls mit der Anzeige im Meßgerät 8 zu integrieren. Dadurch ist im Inneren des Verpackungssystems lediglich die Anordnung des eigentlichen Feuchtesensors 3 erforderlich, während alle Mittel zur Energiebereitstellung, Signalverarbeitung und Anzeige sich jederzeit zugänglich außen befinden.

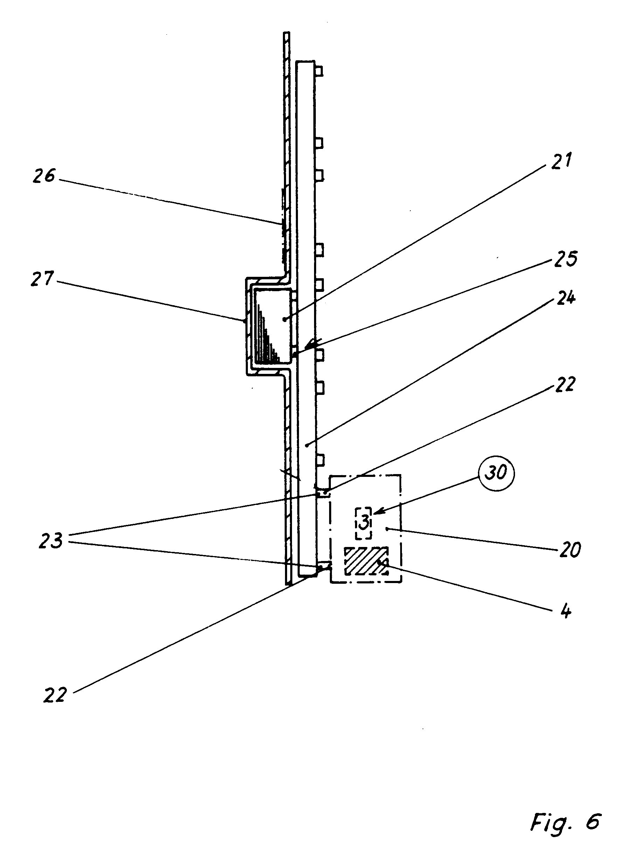

Das Gehäuse oder Behältnis kann z.B. durch Container jeglicher Größe gebildet werden, z.B. die in Lagerhäusern oder Schiffen übereinandergestapelt angeordnet sind. Die Belüftung solcher Vielzahl von Containern mittels einer Luftkühl- und/oder Heizanlage ist aus der DE-PS-19 53 866 oder der DE-OS 37 40 608 bekannt. Eine schematische Querschnittsansicht derselben ist in Fig. 6 gezeigt, wobei der Übersichtlichkeit halber nur der unterste Container 20 in gestrichelter Form angedeutet ist. Die Fig. 6 zeigt eine Klimaanlage 21 des Standes der Technik, die den Container 20 versorgt, der an der Seite über Kupplungen 22 und Anschlüsse 23 mit einem Luftleitkanal 24 verbunden ist. Die Luftleitkanäle 24 bestehen aus Einzelkanälen (nicht gezeigt) und sind mit der Klimaanlage über entsprechende Kupplungen 25 verbunden. Mit 26 ist in der Fig. 6 das Querschott eines nicht gezeigten Schiffes angedeutet. Die Klimaanlage 21 befindet sich in einer Nische 27 des Querschotts 26. Es dürfte einleuchten, daß ein oder auch mehrere Klimaanlagen 21 eingesetzt werden können.
Von besonderer erfindungsgemäßer Bedeutung ist jedoch die Tatsache, daß der Feuchtesensor 3 in einem, mehreren oder allen Containern 20 angeordnet ist.
Letzteres ist durch den Pfeil 30 angedeutet. Der Feuchtesensor 3 mißt die ein feuchteempfindliches, gelagertes und im Container 20 transportiertes Produkt (gestrichelt gezeigt) umgebende Gas- oder Luftfeuchte auf elektrischem Wege, wobei die elektrischen Anschlüsse (nicht gezeigt, z.B. 5 nach Fig. 1 bzw. 2) mittels Bohrungen (nicht gezeigt, z.B. 6 und 7 nach Fig. 1 bzw. 2) durch die Wand des Containers 20 nach außen geführt und dort an ein Meßgerät (nicht gezeigt, z.B. 8 nach Fig. 1 bzw. 2) weitergegeben werden, das die Information akustisch und/oder optisch anzeigt und gleichzeitig eine Stromversorgung zur Lieferung der für den Feuchtesensor 3 erforderlichen Versorgungsspannung aufweist. Ein Öffnen des Containers entfällt also.

Das aus dem System nach außen geführte elektrische Signal kann dann erfindungsgemäß zur Anzeige gebracht oder selbst zu Steuer- und Regelzwecken eingesetzt werden.

## Patentansprüche

1. Anordnung zur Überwachung der Umgebungsfeuchte von in einem geschlossenen System, bestehend aus einer äußeren Umhüllung oder Gehäuse, gelagerten oder transportierten Produkten, wobei ein feuchteempfindliches Sensorelement im Inneren des Systems die dortige Luftfeuchtigkeit erfaßt und in ein äquivalentes elektrisches Signal umwandelt, dieses Signal aus dem System nach außen geführt und außerhalb des Systems zur Anzeige gebracht wird, dadurch gekennzeichnet,
daß das System aus der äußeren Umhüllung oder Gehäuse und einer inneren Verpackungsfolie besteht, und daß das Sensorelement im Inneren oder an der Innenseite der Verpackungsfolie befestigt ist und seine elektrischen Anschlüsse durch die Folie nach außen geführt sind, und daß das nach außen geführte Signal zur Steuer- und Regelung der Umgebungsfeuchtigkeit der produkte eingesetzt oder in ein Fernübertragungsnetz eingespeist wird und zu einer Fernanzeige führt.

2. Anordnung nach Anspruch 1, dadurch gekennzeichnet, daß die Verpackungsfolie aus Polyethylen oder Aluminium besteht.

3. Verfahren nach Anspruch 1, dadurch gekennzeichnet, daß das System durch einen oder mehrere miteinander über einen Kühl- oder Heizstab verbundene Container gebildet wird.

4. Verfahren nach Anspruch 1 bis 3, dadurch gekennzeichnet, daß der bzw. die Container mit der Verpackungsfolie ausgelegt sind.

5. Verfahren nach Anspruch 1, dadurch gekennzeichnet, daß eine Feuchtigkeits-Anzeige an mindestens einer Außenseite der äußeren Umhüllung erfolgt.

6. Verfahren nach Anspruch 5, dadurch gekennzeichnet, daß daß die Anzeige kontinuierlich erfolgt.

7. Verfahren nach Anspruch 5, dadurch gekennzeichnet, daß daß die Anzeige optisch und/oder akustisch den erreichten kritischen Schwellwert der Luftfeuchtigkeit im Inneren signalisiert.

## Claims

1. Means for monitoring the ambient humidity of products stored or transported in a closed system, comprising an outer envelope or casing, a humidity-sensitive sensor element in the interior of the system detecting the atmospheric humidity therein and transforming same into an equivalent electrical signal, said signal is passed to the outside from the system and is displayed outside the system, characterized in that they system comprises the outer envelope or casing and an inner packaging foil or film and that the sensor element is fixed in the interior or to the inside of the packaging foil or film and its electrical connections are passed outwards through the foil or film and that the signal passed to the outside is used for the control and regulation of the ambient humidity of the products, or is fed into a remote transmission network and leads to a remote display.

2. Means according to claim 1, characterized in that the packaging film or foil is made from polyethylene or aluminium.

3. Process according to claim 1, characterized in that the system is formed by one or more containers interconnected by means of a cooling or heating rod.

4. Process according to claim 1 to 3, characterized in that the container or containers are designed with the packaging film or foil.

5. Process according to claim 1, characterized in that a moisture display takes place on at least one outside of the outer envelope.

6. Process according to claim 5, characterized in that the display takes place continuously.

7. Process according to claim 5, characterized in that the display optically and/or acoustically indicates the attained critical threshold of the atmospheric humidity in the interior.

## Revendications

1. Dispositif pour contrôler l'humidité ambiante de produits stockés ou transportés dans un système fermé constitué par une enveloppe extérieure ou un bâti, un élément détecteur sensible à l'humidité captant à l'intérieur du système l'humidité de l'air qui s'y trouve et la transformant en un signal électrique équivalent, ce signal étant conduit du système vers l'extérieur et étant amené hors du système pour affichage, caractérisé en ce que le système est constitué par une enveloppe extérieure ou un bâti et par une feuille d'emballage intérieure et que l'élément détecteur est fixé à l'intérieur ou sur la face intérieure de la feuille d'emballage et ses raccords électriques traversent la feuille vers l'extérieur et que le signal conduit vers l'extérieur est utilisé pour la commande et la régulation de l'humidité ambiante des produits ou est entré dans un réseau de transport d'énergie et mène à un téléaffichage.

2. Dispositif selon la revendication 1, caractérisé en ce que la feuille d'emballage est en polyéthylène ou en aluminium.

3. Dispositif selon la revendication 1, caractérisé en ce que le système est formé par un ou plusieurs conteneurs reliés l'un à l'autre par une cartouche refroidissante ou chauffante.

4. Procédé selon les revendications 1 à 3, caractérisé en ce que le ou les conteneurs sont garnis de feuille d'emballage.

5. Procédé selon la revendication 1, caractérisé en ce qu'un affichage de l'humidité est effectué sur au moins une face extérieure de l'enveloppe extérieure.

6. Procédé selon la revendication 5, caractérisé en ce que l'affichage est effectué en continu.

7. Procédé selon la revendication 5, caractérisé en ce que l'affichage signalise de manière visuelle et/ou acoustique le seuil critique de l'humidité de l'air atteint à l'intérieur.
